# EUROPÄISCHE PATENTANMELDUNG

(11) **EP 4 596 648 A1**
(43) Veröffentlichungstag der Anmeldung: **06.08.2025**
(21) Anmeldenummer: 24155753.7
(22) Anmeldetag: 05.02.2024
(51) Int. Cl.: C09J 7/10, C09J 9/02, C09J 11/02, A61L 15/58, A61B 5/259

(54) **HAFTKLEBSTOFF**

(71) Anmelder: Lohmann GmbH & Co. KG, 56567 Neuwied (DE)
(72) Erfinder: Gärtner, Daniel, 56076 Koblenz (DE)

(57) **Zusammenfassung**

Die Erfindung geht aus von einem Haftklebstoff, insbesondere für Hautanwendungen, mit einem Polymer-Netzwerk, welches zumindest ein Copolymer aufweist.

Um eine Herstellung des Haftklebstoffs und eine Verwendung des Haftklebstoffs für Biosignalmessungen zu verbessern, wird vorgeschlagen, dass der Haftklebstoff 1 bis 15 Gew.-% zumindest eines Weichmachers, 0,5 bis 8 Gew.-% zumindest einer ionischen Verbindung und 5 bis 30°Gew.-% zumindest einer Art von elektrisch leitenden Partikeln aufweist.

## Beschreibung

Die Erfindung betrifft einen Haftklebstoff nach dem Anspruch 1.

Das Anwendungsspektrum für elektrisch leitende Klebstoffe wird stetig größer, entsprechend steigen auch die Anforderungen, welche an solche Klebstoffe gestellt werde. Aufgrund der steigenden Nachfrage nach Elektronik, welche über lange Zeit direkt auf der Haut angebracht werden kann, um beispielsweise Biosignale zu messen, besteht ein Bedürfnis nach elektrisch leitenden Haftklebstoffen, welche sowohl auf unebenen, niederenergetischen Oberflächen wie der Haut als auch auf ebenen, hochenergetischen Oberflächen wie Metallen über lange Zeit eine ausreichende Haftung bereitstellen und im Idealfall rückstandslos und ohne Hautirritationen entfernbar sind.

WO 2020/178217 A1 befasst sich mit elektrisch leitenden Hautklebstoffen, welche eine ionische Flüssigkeit aufweisen, um eine ausreichende elektrische Leitfähigkeit zu gewährleisten.

Die Aufgabe der Erfindung besteht insbesondere darin, einen gattungsgemäßen Haftklebstoff bereitzustellen, welcher verbesserte Eigenschaften bezüglich einer Herstellung und einer Verwendung für Biosignalmessungen aufweist. Die Aufgabe wird erfindungsgemäß durch die Merkmale des Anspruchs 1 gelöst, während vorteilhafte Ausgestaltungen und Weiterbildungen der Erfindung den Unteransprüchen entnommen werden können.

Die Erfindung geht aus von einem Haftklebstoff, insbesondere für Biosignalmessungen, mit einem Polymer-Netzwerk, welches zumindest ein Copolymer aufweist.

Es wird vorgeschlagen, dass der Haftklebstoff 1 bis 15 Gew.-%, vorteilhaft 5 bis 12 Gew.-%, zumindest eines Weichmachers, 0,5 bis 8 Gew.-%, vorteilhaft 1 bis 5 Gew.-%, zumindest einer ionischen Verbindung und 5 bis 30°Gew.-%, vorteilhaft 10 bis 25 Gew.-%, besonders vorteilhaft 15 bis 20 Gew.-%, zumindest einer Art von elektrisch leitenden Partikeln aufweist. Hierdurch können eine Langzeithaftung des Haftklebstoffs und eine Biosignalmessung durch den Haftklebstoff verbessert werden. Tests haben ergeben, dass mehrere Faktoren dafür entscheidend sind, die Dauer der Haftung auf Haut zu maximieren und über diese Dauer einen konsistenten und ausreichenden Kontakt zwischen Elektrode und Haut zu gewährleisten. Insbesondere wurde festgestellt, dass durch die Verwendung elektrisch leitender Partikel zwar der elektrische Widerstand durch den Klebstoff minimiert werden kann, aber Biosignalmessungen durch die Impedanz der Haut weiterhin negativ beeinflusst werden. Ionische Verbindungen sind besser für die Kontaktierung der Haut geeignet, können aber den elektrischen Widerstand des Klebstoffs nicht im selben Maße senken wie elektrisch leitende Partikel. Dies führte zu der Erkenntnis, dass ionische Verbindungen eine bessere Hautkontaktierung erlauben, weil die Leitung durch die Haut auf der Bewegung von Ionen, und nicht der von Elektronen, basiert. Es wurde daher zunächst erwartet, dass eine Kombination beider Lösungen eine optimale Biosignalmessung erlaubt. Allerdings waren die Testresultate dieser Haftklebstoffe weiterhin nicht zufriedenstellend. Diese Resultate führten letztlich zu der überraschenden Erkenntnis, dass aufgrund der Unebenheit der Haut herkömmliche Haftklebstoffe Probleme haben, die Haut ausreichend zu benetzen, was die Biosignalmessung negativ beeinflusst. Dieser Effekt lässt sich auch nicht durch eine Erhöhung des Anteils der ionischen Verbindungen und/oder elektrisch leitenden Partikel kompensieren, im Gegenteil, eine Erhöhung des Anteils der Additive verringert die Haftung des Klebstoffs und reduziert damit gerade bei Langzeitanwendungen die effektive Kontaktfläche noch mehr. Es wurde festgestellt, dass die Benetzung der Haut durch den Haftklebstoff durch das Hinzufügen von Weichmachern verbessert werden kann. Basierend auf all diesen Erkenntnissen wurden innerhalb von Testreihen die Anteile von Weichmachern, ionischen Verbindungen und elektrisch leitenden Partikeln variiert, um zu einem Mischungsverhältnis zu gelangen, in welchem die Vorteile der einzelnen Additive voll genutzt werden können, ohne dass die Herstellungskosten zu stark erhöht beziehungsweise die Langzeithaftung des Haftklebstoffs zu stark reduziert werden.

Die angegebenen Gewichtsanteile bezogen auf den Haftklebstoff beziehen sich auf einen Anteil der jeweiligen Substanz an dem Haftklebstoff in einem unvernetzten Zustand. In dem unvernetzten Zustand liegt der Haftklebstoff in Form einer Haftklebmasse vor, welche dazu vorgesehen ist, zur Herstellung einer Haftklebstoffschicht beschichtet zu werden und entweder vor oder nach der Beschichtung eine Polymerisationsreaktion durchläuft.

Die Formulierungen "X bis Y", "mindestens X", "höchstens X" sollen in diesem Zusammenhang so verstanden werden, dass die Grenzen des jeweiligen Wertebereichs eingeschlossen sind.

Unter einem "Haftklebstoff' soll ein Klebstoff verstanden werden, welcher bei 25°C und ohne einen vorhergehenden Aktivierungsschritt, wie beispielsweise einer Erwärmung oder Bestrahlung, permanent haftklebrig ist. Vorzugsweise unterscheidet sich der Klebstoff von strukturellen Klebstoffen, welche dazu vorgesehen sind, nach einem Applizieren auszuhärten, wobei die strukturellen Klebstoffe vor dem Aushärten keine oder nur eine geringe Haftung aufweisen.

Unter einem "Polymer-Netzwerk" soll eine übergeordnete Struktur verstanden werden, welche aus miteinander verknüpften Polymeren besteht. Es wäre denkbar, dass das Polymer-Netzwerk mehrere, zueinander unterschiedlich ausgebildete, Copolymere aufweist. Bevorzugt weist das Polymer-Netzwerk genau eine Art von Copolymer auf. Unter einem "Copolymer" soll ein Polymer verstanden werden, welches zumindest zwei voneinander verschiedene Monomere aufweist. Das Copolymer kann insbesondere als ein statistisches Copolymer, ein Block-Copolymer oder als ein Homo-Polymer ausgebildet sein. Beispielsweise könnte das Copolymer als ein Polyurethan-Copolymer, ein Synthesekautschuk-Copolymer, ein Naturkautschuk-Copolymer, ein Silikon-Copolymer, ein Acrylnitril-Butadien-Styrol-Copolymer, ein Styrol-Acrylnitril-Copolymer oder ein Styrol-Ethylen-Butylen-Styrol-Copolymer ausgebildet sein. Vorteilhaft besteht der Haftklebstoff zu mindestens 50 Gew.-%, besonders vorteilhaft zu mindestens 60 Gew.- % aus Bestandteilen des Polymer-Netzwerks.

Vorteilhaft ist der Haftklebstoff biokompatibel. Darunter, dass der Haftklebstoff "biokompatibel" ist, soll verstanden werden, dass der Haftklebstoff genormte Prüfungen zur Biokompatibilität von Stoffen besteht. Die genormten Prüfungen sind hierbei von einem Land, in welchem die Trockenelektrodenvorrichtung Anwendung findet, abhängig, bevorzugt besteht die Klebstoffschicht zumindest eine Prüfung auf Hautirritation gemäß ISO 10993-10, eine Prüfung auf Hautsensibilisierung gemäß ISO 10993-10 und eine Prüfung auf Zytotoxizität gemäß ISO 10993-5.

Bevorzugt weist der Haftklebstoff bei 25°C und bei einer Impedanzmessung senkrecht durch eine Haftklebstoffschicht, welche 10 bis 100µm dick ist, wobei das Signal 10 Hz beträgt, eine Klebstoffimpedanz Z_{Klebstoff} von höchstens 50 Ω, besonders bevorzugt höchstens 25 Ω, insbesondere höchstens 10 Ω auf. Besonders bevorzugt beträgt die Gesamtimpedanz Z_{Messung}, welche sich aus Klebstoffimpedanz und Hautimpedanz zusammensetzt, bei 25°Cund bei einer Impedanzmessung zwischen zwei Elektroden, welche jeweils auf einer 10 bis 100 µm dicken Haftklebestoffschicht angeordnet sind, wobei die Haftklebstoffschichten beabstandet auf der Haut eines Probanden angeordnet sind, wobei das Signal 10 Hz beträgt, höchstens 10⁶Ω, bevorzugt höchstens 10⁵Ω.

Der Haftklebstoff kann insbesondere beliebige Weichmacher aufweisen. Unter einem "Weichmacher" soll ein Additiv verstanden werden, welches dazu vorgesehen ist, eine Beweglichkeit des Polymer-Netzwerks zu erhöhen, wodurch ein Benetzen von Oberflächen durch den Haftklebstoff erleichtert wird. Die Beweglichkeit des Polymer-Netzwerk spiegelt sich in der Glasübergangstemperatur des Haftklebstoffs wider, welche durch den Weichmacher reduziert wird. Weichmacher sollen im Kontext dieser Erfindung so verstanden werden, dass sie kein Teil des Polymer-Netzwerks sind und die Beweglichkeit lediglich durch physikalische Interaktionen mit dem Polymer-Netzwerk erhöhen, wie beispielsweise Dipol-Interaktionen. Die Weichmacher können beispielsweise Citronensäure-basierte Weichmacher wie Citronensäuretriethylester, Adipinsäure-basierte Weichmacher wie Diethylhexyladipat, Paraffin-basierte Weichmacher wie Paraffinöl, Alkohol-basierte Weichmacher wie Glycerintriacetat, Polyurethane, Polyester und/oder epoxidierte Pflanzenöle aufweisen.

Unter "Additiven" sollen Substanzen verstanden werden, welche nicht Teil der Substanzen sind, welche dazu vorgesehen sind, nach der Polymerisationsreaktion gemeinsam das Polymer-Netzwerk auszubilden und verschiedene zusätzliche Funktionen bereitstellen können. Additive können beispielsweise Initiatoren, Vernetzer, Tackifier, Alterungsschutzmittel, Reaktionsverdünner, Füllmittel wie beispielsweise Glaskugeln oder Keramikkugeln, Schäumungsmittel oder weitere, dem Fachmann bekannte, Additive aufweisen. Im Fall von Initiatoren und Vernetzern können Reste des Additivs nach der Polymerisationsreaktion im Polymer-Netzwerk integriert sein, machen aber nur einen verschwindend geringen Anteil des gesamten Polymer-Netzwerks aus.

Der Haftklebstoff kann insbesondere beliebige ionische Verbindungen aufweisen. Unter einer "ionischen Verbindung" soll ein Additiv verstanden werden, welches dazu vorgesehen ist, in Lösung in Kationen und Anionen zu zerfallen, welche beweglich sind und hierdurch eine elektrische Leitung bereitstellen können. Ionische Verbindungen können in ionische Flüssigkeiten und ionische Polymere unterteilt werden. Unter "ionischen Flüssigkeiten" sollen Salze verstanden werden, deren Schmelztemperatur weniger als 100°C beträgt, wodurch die Kristallstruktur dieser Salze leicht aufgebrochen und die Kationen und Anionen leicht beweglich gemacht werden können.

Zu möglichen Kationen für ionische Flüssigkeiten gehören beispielsweise Pyridinium, Ammonium und Phosphonium. Zu möglichen Anionen für ionische Flüssigkeiten gehören beispielsweise Halogenide, Tetrafluoroborate, Hexafluorophosphate, Trifluoracetate, Triflate und Tosylate. Unter "ionischen Polymeren" sollen Polymere verstanden werden, welche zu mindestens 50 Gew.-%, vorteilhaft mindestens 70 Gew.- %, besonders vorteilhaft vollständig aus Untereinheiten bestehen, die in Lösung entweder Anionen oder Kationen freigeben. Beispielhafte ionische Polymere sind Polystyrolsulfonsäure und dessen Salze, Polyacrylsäure und dessen Salze, Polyalginate, Polyligninsulfonate, Polysaccharide, Polycarbonsäuren, Acrylamid-Copolymere, Polyethylenimin und Polyvinylamin.

Der Haftklebstoff kann insbesondere beliebige elektrisch leitende Partikel aufweisen. Unter "elektrisch leitenden Partikeln" sollen Objekte mit einem durchschnittlichen Durchmesser im Millimeterbereich, Mikrometerbereich oder Nanometerbereich verstanden werden, welche zumindest ein elektrisch leitendes Material aufweisen. Der durchschnittliche Durchmesser, auch "lateral size" genannt, bezeichnet eine Herstellerangabe, welche die durchschnittliche Partikelgröße definiert. "Elektrisch leitende Materialien" sollen in diesem Zusammenhang als sämtliche Materialien, welche bei 25°C eine Leitfähigkeit von mindestens 10⁶ S/m aufweisen, wie insbesondere Metalle, leitfähige Polymere und Kohlenstoff, verstanden werden. Es wäre möglich, dass die elektrisch leitenden Partikel vollständig aus elektrisch leitenden Materialien bestehen. Alternativ könnten elektrisch leitenden Partikel eine Beschichtung aus einem oder mehreren elektrisch leitenden Materialien aufweisen. Beispielsweise könnten die elektrisch leitenden Partikel aus einem elektrisch leitfähigen Polymer wie PEDOT: PSS bestehen. Alternativ könnten die elektrisch leitenden Partikel einen Kunststoff-, Aluminium-, Keramik- oder Glaskern aufweisen, welcher mit Kupfer, Silber oder anderen Metallen beschichtet ist.

Um eine Langzeithaftung des Haftklebstoffs zu steigern, wird vorgeschlagen, dass das Copolymer als ein Acrylatcopolymer ausgebildet ist. Insbesondere sind die Monomere des Copolymers als Acrylatmonomere ausgebildet. Die Begriffe "Acrylatcopolymer" und "Acrylatmonomer" sollen so verstanden werden, dass sie reine Acrylatcopolymere und Acrylatmonomere sowie Methacrylatcopolymere und Methacrylatmonomere umfassen. Insbesondere können beliebige, aus dem Stand der Technik bekannte, Acrylatmonomere verwendet werden, wie beispielsweise Acrylsäure, Methylacrylat, Ethylacrylat, n-Butylacylat, iso-Butylacrylat, tert-Butylacrylat, 2-Ethylhexylacrylat, 2-Hydroxyehtylacrylat, 2-Hydroxypropylacrylat, 2-Hydroxyethylacrylat, isoBornylacrylat, N,N-Dimethylaminoethylacrylat, Laurylacrylat, Stearylacrylat, Benzylacrylat, Methacrylsäure, Methylmethacrylat, Ethylmethacrylat, Isopropylacrylat, n-Butylmethacrylat, iso-Butylmetharcylat, tert-Butylmethacrylat und/oder Cyclohexylmethacrylat. Bevorzugt ist die Säure als Acrylsäure ausgebildet. Besonders bevorzugt ist Acrylsäure das einzige polare Acrylatmonomer des Acrylatcopolymers. Hierdurch kann eine Langzeithaftung des Haftklebstoffs, insbesondere bei Hautanwendungen, verbessert werden. Tests haben ergeben, dass Acrylat-Haftklebstoffe für Langzeitanwendungen auf Hautoberflächen optimal sind, da sie biokompatibel sind, eine ausreichende Atmungsaktivität aufweisen und sowohl rückstandslos als auch schmerzfrei entfernbar sind.

Vorteilhaft besteht das Copolymer zu mindestens 50 Gew.-%, vorteilhaft mindestens 85 Gew.-%, besonders vorteilhaft mindestens 90 Gew.-% aus unpolaren Säureestern, deren molare Massen mindestens 100 g/mol betragen, und zu 0,1 bis 15 Gew.-%, vorteilhaft 1 bis 10 Gew.-%, besonders vorteilhaft 3 bis 7 Gew.-% aus polaren Säuren. Beispielsweise können die Säureester n-Butylacrylat, 2-Ethylhexylacrylat, Isobutylacrylat, Ethylacrylat und/oder Propylacrylat aufweisen. Darunter, dass ein Monomer "polar" ist, soll in diesem Zusammenhang verstanden werden, dass das Monomer zumindest eine polare Gruppe, wie beispielsweise eine OH-Gruppe, aufweist. Darunter, dass ein Monomer "unpolar" ist, soll in diesem Zusammenhang verstanden werden, dass das Monomer frei von polaren Gruppen ist. Vorzugsweise besteht das Copolymer im Wesentlichen aus den polaren und unpolaren Monomeren. "Im Wesentlichen" ist in diesem Zusammenhang so zu verstehen, dass das Copolymer zwar weitere Bestandteile, wie beispielsweise Reste eines Initiators, aufweisen kann, diese aber unter 0,5 Gew.-%, vorteilhaft unter 0,1 Gew.-% des Copolymers ausbilden und die Eigenschaften des Copolymers nicht wesentlich beeinflussen.

Hierdurch können eine Flexibilität des Haftklebstoffs verbessert und sein Anwendungsspektrum erweitert werden. Vorteilhaft kann durch die sorgfältige Einstellung der Polarität des Copolymers eine Atmungsaktivität des Haftklebstoffs optimiert werden. Eine zu geringe Atmungsaktivität führt zu einer Ansammlung von Feuchtigkeit zwischen dem Haftklebstoff und einer Oberfläche, auf welche der Haftklebstoff appliziert ist, wodurch die Langzeithaftung des Haftklebstoffs reduziert wird und bei Hautanwendungen Hautirritationen entstehen. Eine zu hohe Atmungsaktivität führt zu einer Ansammlung von Feuchtigkeit im Haftklebstoff, wodurch die Langzeithaftung ebenfalls reduziert wird. Besonders vorteilhaft kann ein Haftklebstoff mit einer verbesserten Langzeithaftung an Oberflächen, die Feuchtigkeit ausgesetzt sind, wie beispielsweise Fensteroberflächen, Fassadenoberflächen und/oder Hautoberflächen, bereitgestellt werden.

Vorteilhaft kann aufgrund der durchschnittlich hohen Glasübergangstemperatur von Säuren, wobei Acrylsäure beispielsweise eine Glasübergangstemperatur von 100,85°C aufweist, die Glasübergangstemperatur des Copolymers nach oben korrigiert werden. Besonders vorteilhaft kann durch das Einstellen der Glasübergangstemperatur des Copolymers eine Haftklebrigkeit und eine Kohäsion des Haftklebstoffs optimiert werden, wodurch ein vorzeitiges Abfallen des Haftklebstoffs von einer Oberfläche, auf welche der Haftklebstoff appliziert ist, durch einen kohäsiven Bruch und ein Zurückbleiben von Rückständen nach einem Entfernen des Haftklebstoffs vermieden werden kann. Weist der Haftklebstoff eine zu hohe Kohäsion auf, kann er auf unebenen und/oder niederenergetischen Oberflächen eine Benetzung nicht ausreichend bereitstellen. Weist der Haftklebstoff eine zu geringe Kohäsion auf, kann er keine ausreichende Befestigung bereitstellen, wodurch es zu einer teilweisen bis vollständigen Ablösen der Klebeverbindung kommen kann, ferner kann eine zu geringe Kohäsion dazu führen, dass der Haftklebstoff bei einer Entfernung Rückstände zurücklässt.

Das Optimieren einer Eigenschaft des Copolymers mittels einer bestimmten Auswahl von Monomeren soll dahingehend verstanden werden, dass das Copolymer, welches durch eine Polymerisationsreaktion entsteht, andere Eigenschaften aufweist als ein vergleichbares Copolymer, welche durch dieselbe Polymerisationsreaktion entsteht und andere Monomere beziehungsweise andere Gewichtsanteile der Monomere aufweist. Dass die Polymerisationsreaktionen identisch sind, soll dahingehend verstanden werden, dass eine Anzahl an Monomereinheiten, welche sich zur Bildung des Copolymers während der Polymerisationsreaktion zusammenschließen, bei beiden Polymerisationsreaktionen identisch ist. Beispielsweise würde ein Copolymer, welches aus kurzkettigen Monomeren gebildet ist, nach derselben Polymerisationsreaktion zwangsweise leichter sein als ein Copolymer, welches aus langkettigen Monomeren gebildet ist. Dies ist insbesondere bei industriellen Herstellungsverfahren vorteilhaft, deren Anlagen bestimmte Anforderungen an die Polymerisationsreaktion, insbesondere bezüglich einer Reaktionsgeschwindigkeit, stellen, wodurch ein Einstellen der Eigenschaften der hergestellten Copolymere durch ein Anpassen der Polymerisationsreaktion oft nicht möglich ist.

Vorteilhaft beträgt eine mittlere Molmassenverteilung des Copolymers 450.000 bis 900.000 g/mol, besonders vorteilhaft 500.000 bis 800.00 g/mol und besonders bevorzugt 600.000 bis 700.000 g/mol. Besonders vorteilhaft kann durch das Einstellen der mittleren Molmassenverteilung des Copolymers eine Kohäsion und eine Viskosität des Haftklebstoffs optimiert werden. Bevorzugt kann eine Verarbeitungseffizienz des Haftklebestoffs gesteigert werden, insbesondere lässt sich der Haftklebstoff zur Herstellung einer Haftklebstoffschicht mittels gängiger Beschichtungsverfahren, wie beispielsweise dem Vorhangbeschichten, dem Rakelbeschichten oder dem Siebdruck, beschichten. Denkbar wäre auch, dass der Haftklebstoff mittels Tintenstrahldruckverfahren oder 3D-Druckverfahren aufgetragen werden könnte.

Bevorzugt weist der Weichmacher einen Polyether und/oder ein Polyetherpolyol auf. Es wäre denkbar, dass der Weichmacher als eine Mischung aus einem Polyether und/oder einem Polyetherpolyol und weiteren Weichmachern ausgebildet ist. Besonders bevorzugt ist der Weichmacher vollständig als der Polyether und/oder das Polyetherpolyol ausgebildet. Beispielhafte Polyetherpolyole können insbesondere auf den cyclischen Ethern Ethylenoxid, Propylenoxid und/oder Tetrahydrofuran basieren, welche mit Polyolen wie beispielsweise Diolen wie insbesondere Ethylenglycol oder Propylenglycol oder Triolen wie insbesondere Glycerin oder Trimethylpropan polymerisiert sind. Hierdurch kann ein Haftklebstoff bereitgestellt werden, welcher verbesserte Eigenschaften bezüglich einer Herstellung und einer Verwendung für Biosignalmessungen aufweist. Die Fähigkeit eines Weichmachers, eine homogene Masse mit bestimmten Klebstoffen zu bilden, hängt stark von der Polarität des Weichmachers und der Polarität des Polymer-Netzwerks des Klebstoffs ab und lässt sich aufgrund der Komplexität der involvierten Moleküle nur schwer theoretisch vorhersagen. Tests haben ergeben, dass Weichmacher auf Basis von Polyether und/oder Polyetherpolyol sich besonders gut mit Acrylat-basierten Haftklebstoffen, deren Polymer-Netzwerke Copolymere mit einem hohen Anteil an unpolaren Monomeren aufweisen, mischen lassen. Vorteilhaft kann auf den Einsatz von speziellen Verfahren und/oder Lösemitteln zur Homogenisierung der Klebstoffmasse verzichtet werden, wodurch die Herstellungskosten gesenkt und die Umweltfreundlichkeit der Herstellung gesteigert werden können. Besonders vorteilhaft kann eine effektive Wirkung des Weichmachers durch gute Affinität mit dem restlichen Haftklebstoff gesteigert werden, wodurch eine für eine ausreichende Hautkontaktierung notwendige Menge an Weichmacher reduziert werden kann, wodurch nicht nur Herstellungskosten weiter gesenkt, sondern die Langzeithaftung des Haftklebstoffs verbessert werden.

Bevorzugt weist die ionische Verbindung ein Imidazol-Derivat auf. Unter einem "Imidazol-Derivat" soll eine ionische Flüssigkeit verstanden werden, deren Kation ein Imidazolium-Ion ist. Beispiele für Imidazol-Derivate sind 1-Ethyl-3-Methylimidazoliumdicyandiamid, 1-Ethyl-3-Methylimidazoliumchlorid, 1-Ethyl-3-Methylimidazoliumthiocyanat, 1-Ethyl-3-Methylimidazoliumethylsulfat, 1-Ethyl-3-Methylimidazoliumacetat, 1-Ethyl-3-Methylimidazoliummethylsulfat, 1-Ethyl-3-Methylimidazoliumdiethylphosphat und 1-Ethyl-3-Methylimidazoliumtrifluormethansulfonat. Denkbar wäre, dass die ionische Verbindung als eine Mischung aus einem Imidazol-Derivat und anderen ionischen Verbindungen ausgebildet ist. Bevorzugt ist die ionische Verbindung vollständig als das Imidazol-Derivat ausgebildet. Hierdurch kann ein Haftklebstoff bereitgestellt werden, welcher verbesserte Eigenschaften bezüglich einer Herstellung und einer Verwendung für Biosignalmessungen aufweist. Analog zu dem Weichmacher ist auch die Mischbarkeit einer ionischen Verbindung und einem Klebstoff stark von der Polarität der ionischen Verbindung und der Polarität des Polymer-Netzwerks des Haftklebstoffs abhängig. Tests haben ergeben, dass Imidazol-Derivate sich besonders gut mit Acrylat-basierten Haftklebstoffen, deren Polymer-Netzwerke Copolymere mit einem hohen Anteil an unpolaren Monomeren aufweisen, mischen lassen. Vorteilhaft kann auf den Einsatz von speziellen Verfahren und/oder Lösemitteln zur Homogenisierung der Klebstoffmasse verzichtet werden, wodurch die Herstellungskosten gesenkt und die Umweltfreundlichkeit der Herstellung gesteigert werden können. Besonders vorteilhaft kann eine effektive Wirkung der ionischen Verbindung durch gute Affinität mit dem restlichen Haftklebstoff gesteigert werden, wodurch eine für eine ausreichende Hautkontaktierung notwendige Menge an ionischen Verbindungen reduziert werden kann, wodurch nicht nur Herstellungskosten weiter gesenkt, sondern die Langzeithaftung des Haftklebstoffs verbessert werden.

Alternativ oder zusätzlich kann die ionische Verbindung ein PEDOT:PSS-Derivat aufweisen. Denkbar wäre, dass die ionische Verbindung als eine Mischung aus dem PEDOT:PSS-Derivat und anderen Weichmachern, wie einem Imidiazol-Derivat, ausgebildet ist. Bevorzugt ist die ionische Verbindung entweder vollständig als das Imidiazol-Derivat oder vollständig als das PEDOT:PSS-Derivat ausgebildet. Unter einem "PEDOT:PSS-Derivat" soll ein ionisches Polymer verstanden werden, welche aus mindestens zwei verschiedenen Monomeren aufgebaut sind, wobei zumindest eines dieser Monomere auf Polystyrolsulfonat (PSS) basiert und zumindest ein weiteres dieser Monomere auf Poly-3,4-ethylendioxythiopen (PEDOT) basiert. Hierdurch kann ein Haftklebstoff bereitgestellt werden, welcher verbesserte Eigenschaften bezüglich einer Herstellung und einer Verwendung für Biosignalmessungen aufweist. Analog zu Imidiazol-Derivaten haben Tests mit ionischen Polymeren erwiesen, dass PEDOT:PSS-Derivate sich besonders gut mit Acrylat-basierten Haftklebstoffen, deren Polymer-Netzwerke Copolymere mit einem hohen Anteil an unpolaren Monomeren aufweisen, mischen lassen. Der Grund dafür, dass Tests sowohl mit ionischen Flüssigkeiten als auch mit ionischen Polymeren durchgeführt wurden, ist der, dass ionische Flüssigkeiten zwar generell eine höhere elektrische Leitfähigkeit bereitstellen und auf Dispergiermittel zur Mischung mit dem restlichen Haftklebstoff verzichten können, aber aufgrund der hohen Mobilität der Anionen und Kationen der Verdacht besteht, dass diese aus dem Haftklebstoff entweichen und zu Hautschäden führen könnten. Dagegen weisen ionische Polymere aufgrund der Größe ihrer Moleküle kein Entweichungsrisiko auf, haben aber aufgrund des Dispergiermittels, welches zur Mischung mit dem restlichen Haftklebstoff notwendig ist, höhere Herstellungskosten und eine schlechtere Umweltbilanz.

Es wäre denkbar, dass die elektrisch leitenden Partikel als Metallpartikel oder metallbeschichtete Partikel ausgebildet sind. Um eine Konstruktion der Trockenelektrodenvorrichtung weiter zu verbessern, wird vorgeschlagen, dass die elektrisch leitenden Partikel Graphen-Mikroplättchen ausgebildet sind. Unter "Mikroplättchen" sollen schichtförmige Partikel verstanden werden, deren durchschnittlicher Durchmesser mindestens 1 µm, vorteilhaft mindestens 3 µm, und höchstens 20 µm, vorteilhaft höchstens 10 µm, beträgt. Vorteilhaft beträgt eine Dicke der Mikroplättchen mindestens 1 nm, besonders vorteilhaft mindestens 5 nm, bevorzugt höchstens 500 nm, besonders bevorzugt höchstens 250 nm. Hierdurch kann die Verwendung des Haftklebstoffs für Biosignalmessungen weiter verbessert werden. Vorteilhaft kann durch die geringe Dicke und große Fläche der Mikroplättchen ein hoher Einfluss jedes einzelnen Partikels auf die elektrische Leitfähigkeit der Klebstoffschicht gewährleistet werden, wodurch die Menge an elektrisch leitenden Partikeln, die hinzugefügt werden muss, gering gehalten wird. Besonders vorteilhaft kann auf die Verwendung von Nanopartikeln, deren Erstreckung in jede Richtung im Nanometerbereich ist und damit in der Lage sind, in menschliche Zellen einzudringen, verzichtet werden. Darüber hinaus haben Tests gezeigt, dass elektrisch Leitende Partikel, welche Metalle enthalten, entweder keine ausreichende elektrische Leitfähigkeit bereitstellen, oder in so großen Mengen hinzugefügt werden müssen, dass die Langzeithaftung des Haftklebstoffs reduziert wird. Kohlenstoff-basierte Partikel hingegen haben sich durch eine hohe elektrische Leitfähigkeit und eine gute Hautverträglichkeit bewährt. Somit können durch die Verwendung von Graphen-Mikroplättchen die Vorteile einer für Hautanwendungen idealen Form und eines für Hautanwendungen idealen Materials kombiniert werden.

Es wäre denkbar, dass der Haftklebstoff mehr als 10 Gew.-% eines Vernetzers aufweist, um die Kohäsion des Haftklebstoffs zu optimieren. Um Langzeithaftung des Haftklebstoffs weiter zu verbessern, wird vorgeschlagen, dass der Haftklebstoff 0,01 bis 10 Gew.-%, vorteilhaft 0,1 bis 5 Gew.-%, besonders vorteilhaft 0,15 bis 1 Gew.-% an Vernetzern aufweist. Unter einem "Vernetzer" soll ein Additiv verstanden werden, welche dazu vorgesehen ist, nach der Polymerisationsreaktion einzelne Copolymere zur Bildung des Polymer-Netzwerks miteinander zu verknüpfen. Insbesondere unterscheidet sich der Vernetzer von einem "Härter", welcher dazu vorgesehen ist, während der Polymerisationsreaktion die Monomere zu dem Copolymer zu verknüpfen. Der Vernetzer kann insbesondere ein beliebiger bekannter, für Acrylatklebstoffe geeigneter, Vernetzer sein, wie beispielsweise Ethylenglycoldiacrylat, N,N'-Methylenbisacrylamid oder Aluminiumactylacetonat. Es wäre denkbar, dass der Haftklebstoff mehrere verschiedene Vernetzer aufweist. Vorzugsweise weist der Haftklebstoff genau einen Vernetzer auf. Hierdurch kann eine Langzeithaftung des Haftklebstoffs verbessert werden. Vorteilhaft kann insbesondere durch das Einstellen der Kohäsion mittels der Glasübergangstemperatur und der mittleren Molmassenverteilung des Copolymers darauf verzichtet werden, die Kohäsion stattdessen durch Zugabe von Vernetzern einzustellen. Besonders vorteilhaft kann durch eine Reduzierung der benötigten Menge an Vernetzern eine Adhäsion des Haftklebstoffs verbessert werden, da durch den Vernetzer eine Anzahl freier Copolymerketten, welche zum Aufbau einer Haftverbindung beitragen, reduziert wird.

Der Haftklebstoff kann insbesondere zur Herstellung einer Haftklebstoffschicht mit einer Dicke von 10 bis 100 µm, vorteilhaft 20 - 80 µm, besonders vorteilhaft 30 - 60 µm, verwendet werden. Beispielsweise kann die Haftklebstoffschicht als Teil eines Transferklebebands ausgebildet sein. Das Transferklebeband weist die Haftklebstoffschicht und einen oder zwei Release Liner auf. Im Fall, dass das Transferklebeband einen Release Liner aufweist, ist das Transferklebeband vorzugsweise als ein gewickeltes Transferklebeband ausgebildet. Im Fall, dass das Transferklebeband zwei Release Liner aufweist, sind die Release Liner auf gegenüberliegenden Seiten der Haftklebstoffschicht appliziert. Hierdurch kann eine Haftklebstoffschicht mit verbesserten Eigenschaften bezüglich einer Langzeithaftung und einer Flexibilität bereitgestellt werden. Tests haben ergeben, dass eine Dicke der Haftklebstoffschicht in dem genannten Wertebereich optimale Eigenschaften bezüglich einer elektrischen Leitfähigkeit und Langzeithaftung ermöglicht. Haftklebstoffschichten, die zu dünn sind, haben den Nachteil, dass sie leicht reißen und somit einerseits schwer zu verarbeiten sind und andererseits keine robuste Haftung bereitstellen können. Haftklebstoffschichten, die zu dick sind, haben den Nachteil, dass sie höhere Materialkosten sowohl bezüglich des Haftklebstoffs als auch bezüglich der elektrisch leitenden Partikel verursachen und aufgrund ihrer Dicke zu kohäsiven Brüchen neigen, ferner müssten für eine ausreichende elektrische Leitfähigkeit so viele elektrisch leitende Partikel hinzugefügt werden, dass die Langzeithaftung der Haftklebstoffschicht darunter leiden würde.

Der Haftklebstoff kann insbesondere zur Herstellung eines Haftklebebands, aufweisend eine oder zwei der Haftklebstoffschichten und einen elektrisch leitenden Träger, verwendet werden. Insbesondere kann der elektrisch leitende Träger als ein Textil ausgebildet sein und beispielsweise aus Metallfasern, beschichteten Glasfasern, beschichteten Kunststofffasern und/oder elektrisch leitenden Polymerfasern bestehen. Alternativ kann der elektrisch leitende Träger frei von Ausnehmungen und beispielsweise als eine Metallfolie, eine elektrisch leitende Polymerfolie oder eine Kohlenstofffolie ausgebildet sein. Hierdurch kann ein einseitiges oder doppelseitiges Haftklebeband mit verbesserten Eigenschaften bezüglich einer Langzeithaftung und einer Flexibilität bereitgestellt werden.

Weitere Vorteile ergeben sich aus der folgenden Zeichnungsbeschreibung.

Es zeigt:

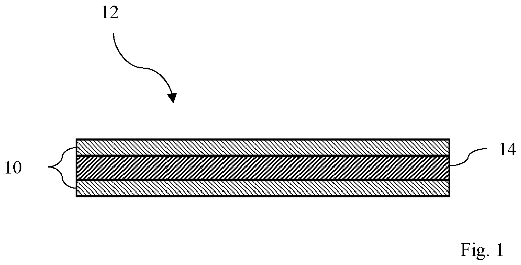

- Fig. 1: eine schematische Darstellung eines Haftklebebands mit einer Haftklebstoffschicht in einer Querschnittsansicht.

Figur 1 zeigt ein Haftklebeband 12. Das Haftklebeband 12 ist als ein doppelseitiges Haftklebeband ausgebildet. Alternativ könnte das Haftklebeband 12 auch als ein einseitiges Haftklebeband ausgebildet sein. Das Haftklebeband 12 weist zwei Haftklebstoffschichten 10 auf. Die Haftklebstoffschichten 10 sind zueinander identisch ausgebildet, weshalb im Folgenden lediglich eine der Haftklebstoffschichten 10 beschrieben wird. Alternativ könnten die Haftklebstoffschichten 10 auch voneinander verschieden ausgebildet sein.

Das Haftklebeband 12 weist einen elektrisch leitenden Träger 14 auf. Der elektrisch leitende Träger 14 ist als eine Aluminiumfolie ausgebildet. Alternativ könnte der elektrisch leitende Träger 14 auch als eine Folie aus einem anderen Metall, aus einem elektrisch leitenden Polymer, aus Kohlenstoff, oder ein Textil aus elektrisch leitenden Fasern sein.

Die Haftklebstoffschicht 10 weist eine Dicke von 30 µm auf. Die Haftklebstoffschicht 10 weist einen Haftklebstoff auf. Die Haftklebstoffschicht 10 ist anisotrop elektrisch leitend.

Der Haftklebstoff weist 12 Gew.-% an Weichmachern auf. Die Weichmacher sind vollständig als ein Polyetherpolyol ausgebildet. Alternativ könnten die Weichmacher auch als eine Mischung aus verschiedenen Arten von Weichmachern ausgebildet sein.

Der Haftklebstoff weist 2,5 Gew.-% an ionischen Verbindungen auf. Die ionischen Verbindungen sind vollständig als ein Imidiazolium-Derivat ausgebildet. Alternativ könnten die ionischen Verbindungen vollständig als ein PEDOT:PSS-Derivat oder als eine Mischung verschiedener Arten von ionischen Verbindungen ausgebildet sein.

Der Haftklebstoff weist 18,5 Gew.-% an elektrisch leitenden Partikeln auf. Die elektrisch leitenden Partikel sind als Graphen-Mikroplättchen ausgebildet. Es wäre auch denkbar, dass die elektrisch leitenden Partikel als Metall-Mikroplättchen oder Kohlenstoff-Mikrokugeln ausgebildet sein könnten. Die Graphen-Mikroplättchen weisen laut Hersteller einen mittleren Durchmesser von 5 µm und eine Dicke von unter 50 nm auf.

Der Haftklebstoff weist ein Polymer-Netzwerk auf. Der Haftklebstoff besteht zu 66,8 Gew.-% aus dem Polymer-Netzwerk. Das Polymer-Netzwerk weist eine Mehrzahl an Acrylatcopolymeren auf. Das Polymer-Netzwerk besteht vollständig aus den Acrylatcopolymeren. Alternativ oder zusätzlich könnte das Polymer-Netzwerk auch ein oder mehrere Polyurethan-Copolymere, Kautschuk-Copolymere und/oder Silikon-Copolymere aufweisen. Das Acrylatcopolymer weist 95 Gew.-% an unpolaren Acrylatmonomeren und 5 Gew.-% an polaren Acrylatmonomeren auf. Das polare Acrylatmonomer ist als Acrylsäure ausgebildet. Das Acrylatcopolymer besteht zu 95 Gew.-% aus Acrylsäureestern, deren mittlere Molmassenverteilung mindestens 100 g/mol beträgt. Eine mittlere Molmassenverteilung des Acrylatcopolymers beträgt 650.000 g/mol.

In der folgenden Tabelle ist eine Zusammensetzung des Acrylatcopolymers dargestellt:

| Substanz | Funktion | Gewichtsanteil |
|---|---|---|
| n-Butylacrylat | Unpolares Acrylatmonomer | 40 Gew.-% |
| 2-Ethylhexylacrylat | Unpolares Acrylatmonomer | 55 Gew.-% |
| Acrylsäure | Polares Acrylatmonomer | 5 Gew.-% |

Der Haftklebstoff weist 0,2 Gew.-% an Vernetzern auf. Der Vernetzer ist als Aluminiumacetylacetonat ausgebildet. Alternativ könnte der Vernetzer auch als ein beliebiger anderer Vernetzer für Acrylat-Haftklebstoffe ausgebildet sein.

### Bezugszeichen

- 10: Haftklebeband
- 12: Haftklebstoffschicht
- 14: Elektrisch leitender Träger

## Patentansprüche

1. Haftklebstoff, insbesondere für Biosignalmessungen, mit einem Polymer-Netzwerk, welches zumindest ein Copolymer aufweist, **gekennzeichnet durch** 1 bis 15 Gew.-% zumindest eines Weichmachers, 0,5 bis 8 Gew.-% zumindest einer ionischen Verbindung und 5 bis 30°Gew.-% zumindest einer Art von elektrisch leitenden Partikeln.

2. Haftklebstoff nach Anspruch 1, **gekennzeichnet durch** 5 bis 12 Gew.-% des Weichmachers.

3. Haftklebstoff nach Anspruch 1 oder 2, **gekennzeichnet durch** 1 bis 5 Gew.- % der ionischen Verbindung.

4. Haftklebstoff nach einem der vorhergehenden Ansprüche, **gekennzeichnet durch** 15 bis 20 Gew.-% der elektrisch leitenden Partikel.

5. Haftklebstoff nach einem der vorhergehenden Ansprüche, **dadurch gekennzeichnet, dass** das Copolymer als ein Acrylatcopolymer ausgebildet ist.

6. Haftklebstoff nach einem der vorhergehenden Ansprüche, **dadurch gekennzeichnet, dass** das Copolymer zu mindestens 50°Gew.-% aus unpolaren Säureestern, deren molare Massen mindestens 100 g/mol betragen und zu 0,1 - 20 Gew.-% aus polaren Säuren besteht.

7. Haftklebstoff nach einem der vorhergehenden Ansprüche, **dadurch gekennzeichnet, dass** der Weichmacher einen Polyether und/oder ein Polyetherpolyol aufweist.

8. Haftklebstoff nach einem der vorhergehenden Ansprüche, **dadurch gekennzeichnet, dass** die ionische Verbindung ein Imidazol-Derivat aufweist.

9. Haftklebstoff nach einem der vorhergehenden Ansprüche, **dadurch gekennzeichnet, dass** die ionische Verbindung ein PEDOT:PSS-Derivat aufweist.

10. Haftklebstoff nach einem der vorhergehenden Ansprüche, **dadurch gekennzeichnet, dass** die elektrisch leitenden Partikel Graphen-Mikroplättchen aufweisen.

11. Haftklebstoff nach einem der vorhergehenden Ansprüche, **gekennzeichnet durch** 0,01 bis 10 Gew.-% an Vernetzern.
